# EUROPÄISCHE PATENTSCHRIFT

(11) **EP 0 328 000 B1**
(45) Veröffentlichungstag und Bekanntmachung des Hinweises auf die Patenterteilung: **18.01.1995**
(21) Anmeldenummer: 89101940.8
(22) Anmeldetag: 03.02.1989
(51) Int. Cl.: C07D 487/14, C07D 487/22, A61K 31/40, A61K 31/495, A61K 31/55

(54) **Indolocarbazol-Derivate, Verfahren zu deren Herstellung und diese enthaltende Arzneimittel**
Indolocarbazole derivatives, process for their preparation and medicines containing same
Dérivés d'indolocarbazole, procédé pour leur préparation et médicaments les contenant

(30) Priorität: 06.02.1988 DE 3803620
(43) Veröffentlichungstag der Anmeldung: 16.08.1989
(73) Patentinhaber: GÖDECKE AKTIENGESELLSCHAFT, D-10587 Berlin (DE)
(72) Erfinder: Kleinschroth, Jürgen, Dr., D-7809 Denzlingen (DE); Hartenstein, Johannes, Dr., D-7801 Stegen-Wittental (DE); Barth, Hubert, Dr., D-7830 Emmendingen (DE); Schächtele, Christoph, Dr., D-7800 Freiburg (DE); Rudolph, Claus, Dr., D-7801 Vörstetten (DE); Weinheimer, Günter, Dr., D-7809 Denzlingen (DE)

(56) Entgegenhaltungen:
- TETRAHEDRON LETTERS, Band 24, Nr.13, 1983, Seiten 1441-1444, Pergamon Press Ltd, Oxford, GB; I.HUGHES et al.: " Synthesis of arcyriaflavin B"
- CHEMICAL ABSTRACTS, Band 107, Nr. 25, 21.Dezember 1987, Seite 796, Zusammenfassung Nr. 236750 y, Columbus, Ohio, US

## Beschreibung

Die Erfindung betrifft neue Indolocarbazol-Derivate der allgemeinen Formel I 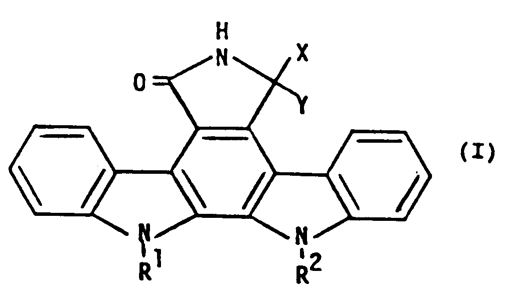
in welcher R¹ und R² gleich oder verschieden sind und Wasserstoff, eine geradkettige oder verzweigte Alkylgruppe mit 1 bis 6 C-Atomen, eine Benzylgruppe, eine unsubstituierte oder substituierte Aminoalkylgruppe mit bis zu 12 C-Atomen, einen Alkoxycarbonylalkylrest mit bis zu 6, bevorzugt mit bis zu 4 C-Atomen, einen Rest -CH₂-CO-NR³R⁴ bei dem R³ und R⁴ gleich oder verschieden sind und für Wasserstoff, eine Alkylgruppe mit 1 bis 4 C-Atomen oder eine Benzylgruppe stehen, oder R¹ und/oder R² einen Halogenalkyl-, Hydroxyalkyl- oder Alkoxyalkylrest mit jeweils bis zu 6 C-Atomen, einen Benzoyloxyalkoxyalkyl-, Acetyloxyalkoxyalkyl- oder Hydroxyalkoxyalkylrest mit jeweils bis zu 11 C-Atomen, eine Acylgruppe mit 1 bis 4 C-Atomen oder R¹ und R² zusammen eine Alkylengruppe mit 2 bis 4 C-Atomen bedeuten, gegebenenfalls substituiert durch Hydroxy, C₁₋₄-Alkoxy oder Amino, X und Y entweder gleich sind und beide jeweils Wasserstoff bedeuten, oder X und Y verschieden sind, wobei einer der Reste X oder Y für Wasserstoff steht und der andere von den beiden Resten eine Hydroxy- oder eine Alkoxygruppe mit bis zu 4 C-Atomen bedeutet,
mit der Maßgabe, daß nicht alle Reste R¹ , R², X und Y gleichzeitig Wasserstoff bedeuten,
sowie deren pharmakologisch unbedenkliche Salze, Verfahren zur Herstellung der Verbindungen I oder von Regioisomerengemischen aus zweien dieser Verbindungen I sowie Arzneimittel mit einem Gehalt an mindestens einer der Verbindungen I.

Die Herstellung der Verbindungen I erfolgt je nach Substitution nach einem der im folgenden beschriebenen Verfahren:
A) Für den Fall, daß X und Y Wasserstoff bedeuten und R¹ und R² gleich sind, jedoch nicht Wasserstoff bedeuten, oder einer der Reste R¹ oder R² für Wasserstoff steht, werden Verbindungen der allgemeinen Formel I erhalten, indem man ein Indolocarbazol der Formel II 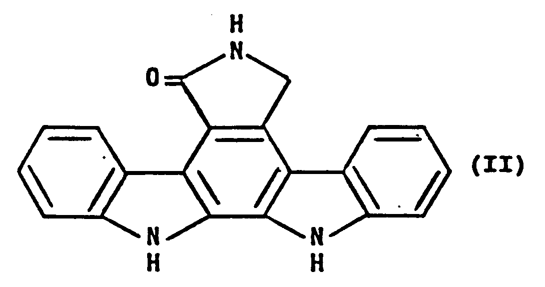 mit einem oder zwei Äquivalenten einer Verbindung der allgemeinen Formel III

   R⁵-Z III

   in der R⁵ für eine geradkettige oder verzweigte Alkylgruppe mit 1 bis 6 C-Atomen, eine unsubstituierte oder substituierte Aminoalkylgruppe mit bis zu 12 C-Atomen, einen Alkoxycarbonylalkylrest mit bis zu 6, bevorzugt mit bis zu 4 C-Atomen, einen Halogenalkyl- oder Alkoxyalkylrest mit jeweils bis zu 6 C-Atomen, einen Benzoyloxyalkoxyalkyl- oder Acetyloxyalkoxyalkylrest mit jeweils bis zu 11 C-Atomen oder eine Acylgruppe mit 1 bis 4 C-Atomen und Z vorzugsweise für Halogen, insbesondere Jod, Brom und Chlor, steht,
   in Gegenwart von einem oder zwei Äquivalenten von Basen wie Hydriden, Carbonaten, Hydroxiden, Oxiden oder Alkoxiden der Alkali- oder Erdalkalimetalle, oder von lithiumorganischen Verbindungen, in an sich bekannter Weise an einem oder beiden Indolstickstoffatomen alkyliert oder acyliert, oder indem man bereits eingeführte Rest R⁵ durch übliche Methoden der präparativen organischen Chemie (z.B. Houben-Weyl, Methoden der Organischen Chemie, Georg Thieme Verlag, Stuttgart 1966) zu einem der Reste R¹ oder R² modifiziert, z.B. durch Hydrolyse, Etherspaltung, Amidbildung oder Reduktion.
   So werden Verbindungen der allgemeinen Formel I, bei denen R¹ und/oder R² für einen Rest -CH₂-CO-NR³R⁴ stehen, vorzugsweise durch Umsetzung von Verbindungen der allgemeinen Formel I, bei denen R¹ und/oder R² einen Alkoxycarbonylmethylrest bedeuten, durch Umsetzung mit Aminen der Formel HNR³R⁴ erzeugt, bei denen R³ und R⁴ die oben angegebenen Bedeutungen besitzen.
   Verbindungen der allgemeinen Formel I, bei denen R¹ und/oder R² für einen Hydroxyalkylrest stehen, werden vorzugsweise durch Hydrolyse von Verbindungen der allgemeinen Formel I, bei denen R¹ und/oder R² einen Halogenalkylrest, insbesondere einen Bromalkyl- oder Chloralkylrest, bedeuten, oder durch Etherspaltung von Verbindungen der allgemeinen Formel I, bei denen R¹ und/oder R² für einen Alkoxyalkylrest stehen, hergestellt.
   Verbindungen der allgemeinen Formel I, bei denen R¹ und/oder R² für einen Hydroxyalkoxyalkylrest stehen, werden nach bekannten Methoden aus Verbindungen der allgemeinen Formel I, bei denen R¹ und/oder R² für einen Benzoyloxyalkoxyalkyl-oder einen Acetyloxyalkoxyalkylrest stehen, hergestellt. Besonders bevorzugte Hydroxyalkoxyalkylreste sind der 2-Hydroxyethoxymethyl- und der 3-Hydroxypropoxymethylrest.
   Verbindungen der allgemeinen Formel I, bei denen R¹ und/oder R² für einen N,N-disubstituierten 3-Amino-2-hydroxypropylrest stehen, werden vorzugsweise durch Alkylierung des Indolocarbazols der Formel II mit 1,1-disubstituierten 3-Hydroxyazetidiniumhalogeniden (J. Org. Chem. 1968, 523) hergestellt.
   Verbindungen der allgemeinen Formel I, bei denen R¹ und R² zusammen einen Alkylenrest -(CH₂)n- mit n = 2 bis 4 bedeuten, werden durch Umsetzung des Indolocarbazols der Formel II mit zwei Äquivalenten einer der oben angegebenen Basen und einem Äquivalent eines Dihalogenalkans, vorzugsweise eines Dibromalkans, erhalten.
   Verbindungen der allgemeinen Formel I, bei denen R¹ und R² zusammen einen Propylenrest der allgemeinen Formel V 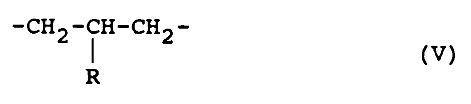 bedeuten, bei dem R für Hydroxy, C₁₋₄-Alkoxy oder Amino steht, werden durch Umsetzung des Indolocarbazols der Formel II mit zwei Äquivalenten einer der oben angegebenen Basen und Epichlorhydrin oder Epibromhydrin erhalten, wobei der zunächst gebildete hydroxysubstituierte Propylenrest nach bekannten Methoden in C₁₋₄-Alkoxy- oder Aminosubstituierte Propylenreste übergeführt werden kann.
   Verbindungen der allgemeinen Formel I, bei denen R¹ und/oder R² für einen Methyl- oder Ethylrest stehen, können auch durch Alkylierung mit Dimethyl- oder Diethylsulfat in bekannter Weise hergestellt werden.
   Das beschriebene Verfahren der Alkylierung oder Acylierung des Indolocarbazols der Formel II in Gegenwart von Basen mit Alkylierungs- oder Acylierungsmitteln ist überraschend, da es nicht absehbar war, daß die Einführung von einem oder zwei Resten R⁵ selektiv an den Indol-Stickstoffatomen und nicht am Stickstoffatom des Lactamrings erfolgt.
B) Für den Fall, daß X und Y Wasserstoff bedeuten, R¹ und R² verschieden sind und weder R¹ noch R² Wasserstoff bedeutet, werden Verbindungen der allgemeinen Formel I durch Umsetzung von Verbindungen der allgemeinen Formel I, die nach Verfahren A) hergestellt wurden, bei denen entweder R¹ oder R² eine der für R⁵ angegebenen Bedeutungen besitzt und der andere der beiden Reste R¹ oder R² für Wasserstoff steht, mit Verbindungen III, bei denen R⁵ eine andere der für R⁵ angegebenen Bedeutungen besitzt, in Gegenwart von Basen in analoger Weise wie bei Verfahren A) hergestellt und gegebenenfalls einer oder beide der Reste mit der Bedeutung von R⁵, wie in Verfahren A) beschrieben, zu einem der Reste R¹ oder R² modifiziert.
C) Verbindungen der allgemeinen Formel I, bei denen X oder Y eine Hydroxygruppe und der andere der beiden Reste Wasserstoff bedeuten, werden durch Reduktion des Imids der Formel IV 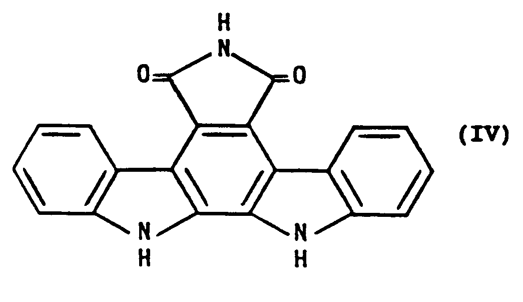 hergestellt. Als bevorzugte Reduktionsmittel werden Zinkamalgam/Chlorwasserstoffgas in C1-C4-Alkoholen oder Zinkamalgam in Eisessig verwendet.
   Verbindungen der allgemeinen Formel I, bei denen X oder Y eine C1-C4-Alkoxygruppe und der andere der beiden Reste Wasserstoff bedeuten, werden entweder durch Reduktion des Imids der Formel IV in C1-C4-Alkoholen, vorzugsweise Zinkamalgam/Chlorwasserstoffgas, oder durch säurekatalysierte Umsetzung von Verbindungen der allgemeinen Formel I, bei denen X oder Y eine Hydroxygruppe und der andere der beiden Reste Wasserstoff bedeuten, mit C1-C4-Alkoholen in wasserfreiem Medium, hergestellt.
   Falls bei der Reduktion mit Zinkamalgam/Chlorwasserstoffgas in C1-C4-Alkoholen Gemische von Verbindungen der allgemeinen Formel I anfallen, bei denen X oder Y entweder eine Hydroxy-oder eine C1-C4-Alkoxygruppe und der andere der beiden Reste Wasserstoff bedeuten, können diese durch übliche Verfahren wie Kristallisation oder Chromatographie getrennt werden.
   Die so erhaltenen Verbindungen der allgemeinen Formel I, bei denen X oder Y eine Hydroxy- oder eine C1-C4-Alkoxygruppe und R¹ und R² Wasserstoff bedeuten, können anschließend gegebenenfalls nach Verfahren A) oder B) zu Verbindungen der allgemeinen Formel I, bei denen X oder Y eine Hydroxy- oder eine C1-C4-Alkoxygruppe und der andere der beiden Reste Wasserstoff und R¹ und R² eine der oben angegebenen Definitionen, jedoch nicht beide gleichzeitig Wasserstoff, bedeuten, alkyliert oder acyliert werden.
   Für R¹ und/oder R² besonders geeignete unsubstituierte oder substituierte Aminoalkylgruppen mit bis zu 12 C-Atomen sind unsubstituierte Aminoalkylgruppen, wie ein 2-Aminoethyl-, ein 3-Aminopropyl- oder ein 1-Amino-2-propylrest, N,N-Dialkylaminoalkyl- oder N,N-Alkylbenzylaminoalkylgruppen mit C1-C4-Alkylsubstituenten an den Stickstoffatomen und 1-4 C-Atomen in der Alkylkette, wobei die Alkylketten durch weitere C1-C4-Alkylreste, C1-C4-Alkoxygruppen oder eine Hydroxygruppe substituiert sein können, insbesondere ein 2-Dimethylaminoethyl-, ein 3-Dimethylamino-1-propyl-, ein 3-Dimethylamino-2-propyl, ein 2-Diethylaminoethyl-, ein 2-[N-Benzyl-N-methylamino]ethyl-, ein 3-[N-Benzyl-N-methylamino]propyl- oder ein 3-Dimethylamino-2-hydroxy-1-propylrest, ein 3-Diethylamino-2-hydroxy-1-propyl-, ein 3-Piperidino-2-hydroxy-1-propyl, ein 3-Dimethylamino-2-methoxy-1-propyl-, ein 3-Diethylamino-2-methoxy-1-propyl-, ein 3-Piperidino-2-methoxy-1-propyl-, ein 3-(N-Benzyl-N-methylamino)-2-methoxy-1-propyl-, ein 3-(N-Benzyl-N-methylamino)-2-hydroxy-1-propyl-, ein 3-(N-Methylamino)-2-methoxy-1-propyl-, ein 3-(N-Methylamino)-2-hydroxy-1-propyl-, ein 4-Dimethylamino-3-methoxy-2-butylrest oder eine 2-Piperidinoethyl-, eine 3-Piperidinopropyl, eine 2-Pyrrolidinoethyl- , eine 3-Pyrrolidinopropyl-, eine 2-Morpholinoethyl-, eine 3-Morpholinopropyl-, eine Pyrrolidin-2-ylmethyl-, eine N-Methyl-pyrrolidin-2-ylmethyl-, eine Piperidin-2-ylmethyl-, eine N-Methyl-piperidin-2-ylmethylgruppe oder eine unsubstituierte oder am Stickstoffatom durch C₁₋₄-Alkyl substituierte Piperazinoalkylgruppe mit 1 bis 4 C-Atomen in der Alkylkette.
   Weitere für R¹ und/oder R² besonders geeignete Reste sind geradkettige oder verzweigte Alkylgruppen mit 1 bis 4 C-Atomen, insbesondere Methyl, Ethyl, n-Propyl, Isopropyl und n-Butyl, Methoxycarbonylmethylgruppen, Benzylgruppen, 2-Methoxyethylgruppen, Acetylgruppen oder auch eine Alkylengruppe mit 2 bis 4 C-Atomen, insbesondere eine Butylengruppe oder eine hydroxysubstituierte Propylengruppe, welche R¹ und R² zusammen bilden.

Verbindungen der allgemeinen Formel I nach Verfahren A), B) oder C), bei denen R¹ und R² verschieden sind, können als Regioisomerengemische verwendet werden. Durch bekannte Trennverfahren wie Kristallisation oder Chromatographie können die beiden Regioisomeren getrennt werden.

Verbindungen der allgemeinen Formel I, die ein chirales Zentrum aufweisen, können als Stereoisomerengemische oder in Form der Enantiomeren verwendet werden. Die Enantiomeren können nach den für optische Trennungen von Stereoisomeren verwendeten üblichen Verfahren erhalten werden.

Basische Verbindungen der allgemeinen Formel I, welche ein basisches Zentrum an R¹ oder R² aufweisen, werden zum Zwecke der Reinigung und aus galenischen Gründen bevorzugt in kristalline, pharmakologisch verträgliche Salze übergeführt. Die Salze werden in üblicher Weise durch Neutralisation der Basen mit entsprechenden anorganischen oder organischen Säuren erhalten.

Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Die Herstellung der als Ausgangsmaterial dienenden Indolocarbazole der Formeln II und IV in der Literatur beschrieben oder kann in analoger Weise durchgeführt werden (Heterocycles 1983, 20, 469; Tetrahedron Letters 1983, 1441).

Die erfindungsgemäßen Verbindungen sind potente Inhibitoren von Proteinkinasen wie der Proteinkinase C. So zeigt z.B. die Verbindung von Beispiel 1a im Enzym-Assay der mit Phosphatidylserin und Diacylglycerol aktivierten Proteinkinase C eine 50 %ige Inhibierung bei einer Konzentration von 66 Nanomol/Liter. Der Versuch wurde gemäß EP-OS-0 255 126 (Hemmung von Proteinkinase C) durchgeführt. Es sind zwar schon Indolocarbazole als Inhibitoren der Proteinkinase C beschrieben worden (J. Antibiot. 1977, 30, 275; Biochem. Biophys. Res. Commun. 1986, 135, 397). Dabei handelt es sich aber hauptsächlich um Indolocarbazol-N,N-glykoside mikrobiellen oder semisynthetischen Ursprungs.

Die Proteinkinase C spielt für die intracelluläre Signaltransduktion eine wichtige Schlüsselrolle und ist eng mit der Regulation von kontraktilen, sekretorischen und proliferativen Prozessen verknüpft. Aufgrund dieser Eigenschaften können die erfindungsgemäßen Verbindungen zur Behandlung von Herz-und Gefäßkrankheiten wie Thrombosen, Arteriosklerose, Hypertension, von Entzündungsprozessen, Allergien, Krebs und bestimmten degenerativen Schäden des Zentralnervensystems verwendet werden. Die Verbindungen können in der jeweils geeigneten Formulierung enteral oder parenteral in Dosen von 1 bis 50 mg/kg verabreicht werden.

### Beispiele

### Beispiel 1

### 6,7,12,13-Tetrahydro-5-oxo-12,13-dipropyl-5H-indolo[2,3-a] pyrrolo-[3,4-c]carbazol

20 mg (0,67 mmol) Natriumhydrid (80%ig in Mineralöl) werden in 10 ml trockenem Dimethylformamid suspendiert und 100 mg (0,32 mmol) 6,7,12,13-Tetrahydro-5-oxo-5H-indolo[2,3-a]pyrrolo[3,4-c] carbazol portionsweise bei Raumtemperatur zugegeben. Nach Abklingen der Gasentwicklung wird 1 Stunde bei Raumtemperatur nachgerührt, dann werden 120 mg (0,71 mmol) n-Propyljodid zugefügt und 16 Stunden bei Raumtemperatur gerührt. Das Lösungsmittel wird im Vakuum abrotiert und der Rückstand an Kieselgel mit Toluol/Ethylacetat 1:1 chromatographiert. Die Fraktion mit dem Rf 0,4 wird isoliert, mit Diisopropylether verrührt. Die gebildeten Kristalle werden abfiltriert.

Man erhält 6,7,12,13-Tetrahydro-5-oxo-12,13-dipropyl-5H-indolo[2,3-a]pyrrolo[3,4-c]carbazol in form beiger Kristalle vom Schmp. 180-185° C (Zers.), Ausbeute 43%.

In analoger Weise erhält man

### 12,13-Diethyl-6,7,12,13-tetrahydro-5-oxo-5H-indolo[2,3-a] pyrrolo-[3,4-c]carbazol (1.a)

Schmp. > 225°C (Zers.) aus Diisopropylether, Ausbeute 32%.

Mit zwei Äquivalenten Natriumhydrid und einem Äquivalent 1,4-Dibrombutan wird in analoger Weise erhalten:

### 12,13-Butano-6,7,12,13-tetrahydro-5-oxo-5H-indolo[2,3-a]pyrrolo[3,4-c]carbazol (1.b)

Schmp. > 300° C aus Diisopropylether, Ausbeute 53%.

In analoger Weise erhält man

### 12,13-Dibenzyl-6,7,12,13-tetrahydro-5-oxo-5H-indolo[2,3-a] pyrrolo-[3,4-c]carbazol (1.c)

Schmp. > 250° C (Zers.) aus Diisopropylether, Ausbeute 33%;

### 12,13-Dioctadecyl-6,7,12,13-tetrahydro-5-oxo-5H-indolo[2,3-a] pyrrolo-[3,4-c]carbazol (1.d)

Schmp. 101-103° C aus Diisopropylether/Ethylacetat, Ausbeute 42%.

### Beispiel 2

### 6,7,12,13-Tetrahydro-5-oxo-12-propyl-5H-indolo[2,3-a] pyrrolo[3,4-c]carbazol und 6,7,12,13-Tetrahydro-5-oxo-13-propyl-5H-indolo[2,3-a] pyrrolo[3,4-c]carbazol

Das nach Umsetzung von 100 mg (0,32 mmol) 6,7,12,13-Tetrahydro-5-oxo-5H-indolo[2,3-a]pyrrolo[3,4-c]carbazol mit 10 mg (0,33 mmol) Natriumhydrid (80%ig in Mineralöl) und 60 mg (0,35 mmol) n-Propyljodid analog Beispiel 1 erhaltene Rohprodukt wird chromatographisch an Kieselgel mit Toluol/Ethylacetat 1:1 getrennt. Die Fraktion mit dem Rf 0,3 wird isoliert, mit Diisopropylether verrührt. Die gebildeten beigen Kristalle werden abfiltriert.

Man erhält ein ca. 3:1-Regioisomerengemisch von 6,7,12,13-Tetrahydro-5-oxo-12-propyl-5H-indolo[2,3-a]pyrrolo[3,4-c] carbazol und 6,7,12,13-Tetrahydro-5-oxo-13-propyl-5H-indolo[2,3-a]pyrrolo[3,4-c]carbazol vom Schmp. ca. 300° C (Zers.), Ausbeute 44%.

In analoger Weise erhält man:
6,7,12,13-Tetrahydro-12-(2-methoxyethyl)-5-oxo-5H-indolo[2,3-a] pyrrolo[3,4-c]carbazol und 6,7,12,13-Tetrahydro-13-(2-methoxyethyl)-5-oxo-5H-indolo[2,3-a]pyrrolo[3,4-c]carbazol (2.a), Schmp. > 250° C (Zers.) aus Diisopropylether, 5:1-Regioisomerengemisch, Ausbeute 26%;
mit einem Äquivalent Natriumhydrid und einem Äquivalent Acetanhydrid nach chromatographischer Trennung der Regioisomeren:
13-Acetyl-6,7,12,13-tetrahydro-5-oxo-5H-indolo[2,3-a] pyrrolo[3,4-c]carbazol (2.b), Schmp. > 315°C (Zers.) aus Diisopropylether, Ausbeute 14%;
6,7,12,13-Tetrahydro-12-octadecyl-5-oxo-5H-indolo[2,3-a] pyrrolo[3,4-c]carbazol und 6,7,12,13-Tetrahydro-13-octadecyl-5-oxo-5H-indolo[2,3-a] pyrrolo[3,4-c]carbazol (2.c), Schmp. 170-185° C aus Diisopropylether/Ethylacetat, Ausbeute 40%, 2:1-Regioisomerengemisch.

### Beispiel 3

### 12-Ethyl-6,7,12,13-tetrahydro-5-oxo-5H-indolo[2,3-a]pyrrolo [3,4-c]carbazol

Das nach Umsetzung von 200 mg (0,64 mmol) 6,7,12,13-Tetrahydro-5-oxo-5H-indolo[2,3-a]pyrrolo[3,4-c]carbazol mit 20 mg (0,66 mmol) Natriumhydrid (80%ig in Mineralöl) und 110 mg (0,71 mmol) Ethyljodid analog Beispiel 1 erhaltene Rohprodukt wird chromatographisch an Kieselgel mit Toluol/Ethylacetat 1:1 getrennt. Die Fraktion mit dem Rf 0,25 wird isoliert, mit Toluol/Ethanol 9:1 verrührt und die gebildeten Kristalle werden abfiltriert. Das erhaltene ca. 3:1-Regioisomerengemisch von 12-Ethyl-6,7,12,13-tetrahydro-5-oxo-5H-indolo[2,3-a]pyrrolo[3,4-c] carbazol und 13-Ethyl-6,7,12,13-tetrahydro-5-oxo-5H-indolo [2,3-a]pyrrolo[3,4-c]carbazol wird mit Aceton ausgekocht und die Kristalle nach dem Abkühlen abfiltriert.

Man erhält 12-Ethyl-6,7,12,13-tetrahydro-5-oxo-5H-indolo[2,3-a] pyrrolo[3,4-c]carbazol in form beiger Kristalle vom Schmp.>290°C (Zers.), Ausbeute 27%.

### Beispiel 4

### 12-(3-Dimethylaminopropyl)-6,7,12,13-tetrahydro-5-oxo-5H-indolo[2,3-a]pyrrolo[3,4-c]carbazol und 13-(3-Dimethylaminopropyl)-6,7,12,13-tetrahydro-5-oxo-5H-indolo[2,3-a]pyrrolo[3,4-c]carbazol

Das nach Umsetzung von 150 mg (0,48 mmol) 6,7,12,13-Tetrahydro-5-oxo-5H-indolo[2,3-a]pyrrolo[3,4-c]carbazol mit 18 mg (0,60 mmol) Natriumhydrid (80%ig in Mineralöl) und 73 mg (0,60 mmol) 3-Dimethylaminopropylchlorid analog Beispiel 1 erhaltene Rohprodukt wird chromatographisch an Kieselgel mit Ethylacetat/Aceton 1:1 getrennt. Die Fraktion mit dem Rf 0,1 wird isoliert, mit Diisopropylether verrührt. Die gebildeten Kristalle werden abfiltriert.

Man erhält ein ca. 6:1-Regioisomerengemisch von 12-(3-Dimethylaminopropyl)-6,7,12,13-tetrahydro-5-oxo-5H-indolo[2,3-a] pyrrolo[3,4-c]carbazol und 13-(3-Dimethylaminopropyl)-6,7,12,13-tetrahydro-5-oxo-5H-indolo[2,3-a]pyrrolo[3,4-c] carbazol in Form beiger Kristalle vom Schmp. 265° C (Zers.), Ausbeute 48%.

In analoger Weise erhält man:
6,7,12,13-Tetrahydro-12-(2-morpholinoethyl)-5-oxo-5H-indolo[2,3-a]pyrrolo[3,4-c]carbazol und 6,7,12,13-Tetrahydro-13-(2-morpholinoethyl)-5-oxo-5H-indolo-[2,3-a]pyrrolo[3,4-c]carbazol (4.a), Schmp. > 230° C (Zers.) aus Diisopropylether, 3:1-Regioisomerengemisch, Ausbeute 30%;
6,7,12,13-Tetrahydro-5-oxo-12-(2-pyrrolidinoethyl)-5H-indolo[2,3-a]pyrrolo[3,4-c]carbazol und 6,7,12,13-Tetrahydro-5-oxo-13-(2-pyrrolidinoethyl)-5H-indolo-[2,3-a]pyrrolo[3,4-c]carbazol (4.b), Schmp. 239-243° C aus Diisopropylether/Ethylacetat, Ausbeute 28%;
(±)-12-(3-Diethylamino-2-methoxy-1-propyl)-6,7,12,13-tetrahydro-5-oxo-5H-indolo[2,3-a]pyrrolo[3,4-c]carbazol und (±)-13-(3-Diethylamino-2-methoxy-1-propyl)-6,7,12,13-tetrahydro-5-oxo-5H- indolo-[2,3-a]pyrrolo[3,4-c]carbazol (4.c), Schmp. 240-260° C (Zers.) aus Diisopropylether/Ethylacetat, Ausbeute 54%, 3:1-Regioisomerengemisch;
(±)-12-(3-Dimethylamino-2-methoxy-1-propyl)-6,7,12,13-tetrahydro-5-oxo-5H-indolo[2,3-a]pyrrolo[3,4-c]carbazol und (±)-13-(3-Dimethylamino-2-methoxy-1-propyl)-6,7,12,13-tetrahydro-5-oxo-5H-indolo[2,3-a]pyrrolo[3,4-c]carbazol (4.d), Schmp. > 190° C (Zers.) aus Diisopropylether/Ethylacetat, 7:3-Regioisomerengemisch, Ausbeute 21%;
(±)-12-[3-(N-Benzyl-N-methylamino)-2-methoxy-1-propyl]-6,7,12,13-tetrahydro-5-oxo-5H-indolo[2,3-a]pyrrolo[3,4-c] carbazol und (±)-13-[3-(N-Benzyl-N-methylamino)-2-methoxy-1-propyl]-6,7,12,13-tetrahydro-5-oxo-5H-indolo[2,3-a]pyrrolo[3,4-c]carbazol (4.e), Schmp. > 150° C (Zers.) aus Diisopropylether/Ethylacetat, 5:3-Regioisomerengemisch, Ausbeute 32%;
6,7,12,13-Tetrahydro-5-oxo-12-(3-piperidinopropyl)-5H-indolo-[2,3-a]pyrrolo[3,4-c]carbazol und 6,7,12,13-Tetrahydro-5-oxo-13-(3-piperidinopropyl)-5H-indolo-[2,3-a]pyrrolo[3,4-c]carbazol (4.f), Schmp. > 230° C (Zers.) aus Diisopropylether, 7:4-Regioisomerengemisch, Ausbeute 17%.

### Beispiel 5

### 6,7,12,13-Tetrahydro-12-methyl-5-oxo-5H-indolo[2,3-a] pyrrolo[3,4-c]carbazol und 6,7,12,13-Tetrahydro-13-methyl-5-oxo-5H-indolo[2,3-a] pyrrolo[3,4-c]carbazol

29 mg (0,97 mmol) Natriumhydrid (80% ig in Mineralöl) werden in 20 ml trockenem Dimethylformamid suspendiert und 200 mg (0,64 mmol), 6,7,12,13-Tetrahydro-5-oxo-5H-indolo[2,3-a]pyrrolo[3,4-c] carbazol portionsweise bei Raumtemperatur zugegeben. Nach Abklingen der Gasentwicklung werden 0,09 ml (0,95 mmol) Dimethylsulfat zugefügt und 72 Stunden bei Raumtemperatur gerührt. Das Lösungsmittel wird im Vakuum abdestilliert und der Rückstand mit 20 ml Kaliumcarbonatlösung versetzt. Es wird zweimal mit je 20 ml Ethylacetat extrahiert. Die Ethylacetatlösungen wird über Natriumsulfat getrocknet und anschließend im Vakuum eingedampft. Der Rückstand wird an Kieselgel mit Dichlormethan/Methanol 95:5 chromatographiert. Die Fraktion mit dem Rf 0,4 wird isoliert, mit Diisopropylether/Aceton 9:1 verrührt. Die gebildeten Kristalle werden abfiltriert.

Man erhält ein ca. 7:1-Regioisomerengemisch von 6,7,12,13-Tetrahydro-12-methyl-5-oxo-5H-indolo[2,3-a]pyrrolo[3,4-c] carbazol und 6,7,12,13-Tetrahydro-13-methyl-5-oxo-5H-indolo [2,3-a]pyrrolo[3,4-c]carbazol in Form beiger Kristalle vom Schmp. >300° C (Zers.), Ausbeute 38%.

Mit zwei Äquivalenten Natriumhydrid und Dimethylsulfat wird in analoger Weise erhalten:

### 6,7,12,13-Tetrahydro-12,13-dimethyl-5-oxo-5H-indolo[2,3-a] pyrrolo[3,4-c]carbazol (5.a)

Schmp. 248-251° C aus Diisopropylether, Ausbeute 56%

### Beispiel 6

### 6,7,12,13-Tetrahydro-7-hydroxy-5-oxo-5H-indolo[2,3-a] pyrrolo[3,4-c]carbazol (6.a) und 7-Ethoxy-6,7,12,13-tetrahydro-5-oxo-5H-indolo[2,3-a]pyrrolo [3,4-c]carbazol (6.b)

Zu einer gerührten Suspension von 10 g (153 mmol) Zinkstaub und 1 g (3,7 mmol) Quecksilber(II)chlorid in 10 ml Wasser werden 0,5 ml konz. Salzsäure zugegeben. Nach ca. 5 Minuten wird dekantiert. Dann wird das Zinkamalgam zunächst mit Wasser und anschließend mehrfach mit Ethanol gewaschen. Nach der Zugabe von 30 ml trockenem Ethanol wird mit einem Eisbad gekühlt und 330 mg (1,01 mmol) 6,7,12,13-Tetra-hydro-5,7-dioxo-5H-indolo[2,3-a] pyrrolo[3,4-c]carbazol zugefügt. Dann wird langsam unter weiterer Kühlung während einer Stunde trockenes Chlorwasserstoffgas eingeleitet. Es wird filtriert, eingedampft und der Rückstand an Kieselgel mit Toluol/Ethylacetat 1:1 chromatographiert. Die Fraktion mit dem Rf 0,3 wird isoliert und aus Ethylacetat kristallisiert.

Man erhält blaßbeige Kristalle von 7-Ethoxy-6,7,12,13-tetrahydro-5-oxo-5H-indolo[2,3-a]pyrrolo[3,4-c]carbazol (6.b) vom Schmp. >300° C, Ausbeute 22%.

Die Fraktion mit dem Rf 0,2 wird isoliert und mit Diisopropylether verrührt. Man erhält beige Kristalle von 6,7,12,13-Tetrahydro-7-hydroxy-5-oxo-5H-indolo[2,3-a] pyrrolo[3,4-c]carbazol (6.a) vom Schmp. >300° C, Ausbeute 23%.

### Beispiel 7

### 6,7,12,13-Tetrahydro-12-methoxycarbonylmethyl-5-oxo-5H-indolo[2,3-a]pyrrolo-[3,4-c]carbazol und 6,7,12,13-Tetrahydro-13-methoxycarbonylmethyl-5-oxo-5H-indolo[2,3-a]pyrrolo-[3,4-c]carbazol

Das nach Umsetzung von 150 mg (0,48 mmol) 6,7,12,13-Tetrahydro-5-oxo-5H-indolo[2,3-a]pyrrolo[3,4-c]carbazol mit 18 mg (0,60 mmol) Natriumhydrid (80%ig in Mineralöl) und 115 mg (0,75 mmol) Bromessigsäuremethylester analog Beispiel 1 erhaltene Rohprodukt wird chromatographisch an Kieselgel mit Dichlormethan/Methanol 95:5 getrennt. Die Fraktion mit dem Rf 0,35 wird isoliert, mit Diisopropylether verrührt. Die gebildeten Kristalle werden abfiltriert.

Man erhält ein ca. 3:1-Regioisomerengemisch von 6,7,12,13-Tetrahydro-12-methoxycarbonylmethyl-5-oxo-5H-indolo[2,3-a] pyrrolo-[3,4-c]carbazol und 6,7,12,13-Tetrahydro-13-methoxycarbonylmethyl-5-oxo-5H-indolo[2,3-a]pyrrolo-[3,4-c] carbazol in form beiger Kristalle vom Schmp. >300° C, Ausbeute 51%.

### Beispiel 8

### (±)-6,7,12,13-Tetrahydro-12,13-(2-hydroxypropano)-5-oxo-5H-indolo[2,3-a]pyrrolo-[3,4-c]carbazol

Das nach Umsetzung von 300 mg (0,96 mmol) 6,7,12,13-Tetrahydro-5-oxo-5H-indolo[2,3-a]pyrrolo-[3,4-c]carbazol mit 33 mg (1,1 mmol) Natriumhydrid (80%ig in Mineralöl) und 0,1 ml (1,2 mmol) Epibromhydrin in 30 ml trockenem Dimethylformamid analog Beispiel 1 erhaltene Rohprodukt (Reaktionsdauer 72 h bei Raumtemperatur) wird chromatographisch an Kieselgel mit Cyclohexan/Tetrahydrofuran 1:1 getrennt. Die Fraktion mit dem R_{f} 0,15 in Toluol/Ethylacetat 1:1 wird isoliert, mit Diisopropylether/Methanol verrührt und die gebildeten Kristalle abfiltriert. Man erhält 6,7,12,13-Tetrahydro-12,13-(2-hydroxpropano)-5-oxo-5H-indolo[2,3-a]pyrrolo-[3,4-c]carbazol in Form beiger Kristalle, die sich > 300°C zersetzen, Ausbeute 19%.

### Beispiel 9

### (±)-12-(3-Diethylamino-2-hydroxy-1-propyl)-6,7,12,13-tetrahydro-5-oxo-5H-indolo[2,3-a]pyrrolo[3,4-c]carbazol und (±)-13-(3-Diethylamino-2-hydroxy-1-propyl)-6,7,12,13,-tetrahydro-5-oxo-5H-indolo[2,3-a]pyrrolo[3,4-c]carbazol

Das nach Umsetzung von 500 mg (1,61 mmol) 6,7,12,13-Tetrahydro-5-oxo-5H-indolo[2,3-a]pyrrolo[3,4-c]carbazol mit 58 mg (1,92 mmol) Natriumhydrid (80%ig in Mineralöl) und 447 mg (2,70 mmol) 1,1-Diethyl-3-hydroxyazetidiniumchlorid in 50 ml trockenem Dimethylformamid analog Beispiel 1 (Reaktionsdauer 48 h bei Raumtemperatur) erhaltene Rohprodukt wird zwischen 200 ml Ethylacetat und 50 ml Wasser verteilt. Die organische Phase wird abgetrennt, getrocknet (Natriumsulfat) und eingedampft. Der Rückstand wird an Kieselgel mit Dichlormethan/Methanol 95:5 chromatographisch getrennt. Die Fraktion mit dem R_{f} = 0,1 wird isoliert, mit Diisopropylether/Ethylacetat verrührt und die gebildeten Kristalle abfiltriert.

Man erhält (±)-12-(3-Diethylamino-2-hydroxy-1-propyl)-6,7,12,13-tetrahydro-5-oxo-5H-indolo[2,3-a]pyrrolo[3,4-c]carbazol und (±)-13-(3-Diethylamino-2-hydroxy-1-propyl)-6,7,12,13-tetrahydro-5-oxo-5H-indolo[2,3-a]pyrrolo[3,4-c]carbazol in Form blaßbeiger Kristalle als 1:1-Regioisomerengemisch vom Schmp. > 195°C (Zers.), Ausbeute 44%.

In analoger Weise erhält man:
(±)-12-(3-Piperidino-2-hydroxy-1-propyl)-6,7,12,13-tetrahydro-5-oxo-5H-indolo[2,3-a]pyrrolo[3,4-c]carbazol und (±)-13-(3-Piperidino-2-hydroxy-1-propyl)-6,7,12,13-tetrahydro-5-oxo-5H-indolo[2,3-a]pyrrolo[3,4-c]carbazol (9.a), Schmp. > 205°C (Zers.) aus Diisopropylether, Ausbeute 28%.

### Beispiel 10

### (±)-6,7,12,13-Tetrahydro-12-(3-N-methylamino-2-methoxy-1-propyl)-5-oxo-5H-indolo[2,3-a]pyrrolo-[3,4-c]carbazol und (±)-6,7,12,13-Tetrahydro-13-(3-N-methylamino-2-methoxy-1-propyl)-5-oxo-5H-indolo[2,3-a]pyrrolo-[3,4-c]carbazol

100 mg (0,2 mmol) (±)-12-[3-(N-Benzyl-N-methylamino)-2-methoxy-1-propyl]-6,7,12,13-tetrahydro-5-oxo-5H-indolo[2,3-a]pyrrolo-[3,4-c]carbazol und (±)-13-[3-(N-Benzyl-N-methylamino)-2-methoxy-1-propyl]-6,7,12,13-tetrahydro-5-oxo-5H-indolo[2,3-a]-pyrrolo[3,4-c]carbazol (Beispiel 4.e) in 30 ml Eisessig werden mit 100 mg Palladium auf Aktivkohle (10% an Pd / 50% Wasser) 5 h bei 55-60°C hydriert. Der Katalysator wird abfiltriert und die Lösung im Vakuum eingedampft. Der Rückstand wird zwischen Natriumhydrogencarbonatlösung (50 ml) und Ethylacetat (100 ml) verteilt. Die organische Phase wird abgetrennt, getrocknet (Natriumsulfat) und eingedampft. Der Rückstand wird mit Diisopropylether/Ethylacetat (4:1) verrührt, die Kristalle abfiltriert und getrocknet.

Man erhält (±)-6,7,12,13-Tetrahydro-12-(3-N-methylamino-2-methoxy-1-propyl)-5-oxo-5H-indolo[2,3-a]pyrrolo[3,4-c]carbazol und (±)-6,7,12,13-Tetrahydro-13-(3-N-methylamino-2-methoxy-1-propyl)-5-oxo-5H-indolo[2,3-a]pyrrolo[3,4-c]carbazol in Form blaßbeiger Kristalle vom Schmp. > 170°C (Zers.), Ausbeute 62%, als 3:1-Regioisomerengemisch.

## Patentansprüche

1. Indolocarbazol-Derivate der allgemeinen Formel I 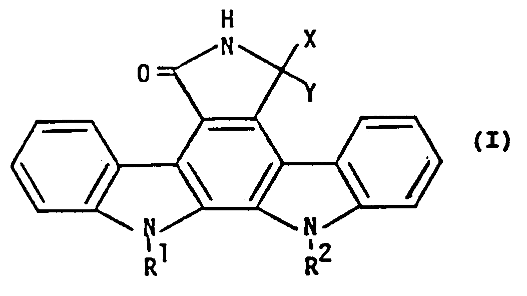 in welcher R¹ und R² gleich oder verschieden sind und Wasserstoff, eine geradkettige oder verzweigte Alkylgruppe mit 1 bis 6 C-Atomen, eine Benzylgruppe, eine unsubstituierte oder substituierte Aminoalkylgruppe mit bis zu 12 C-Atomen, einen Alkoxycarbonylalkylrest mit bis zu 6 C-Atomen, einen Rest -CH₂-CO-NR³R⁴ bei dem R³ und R⁴ gleich oder verschieden sind und für Wasserstoff, eine Alkylgruppe mit 1 bis 4 C-Atomen oder eine Benzylgruppe stehen, oder R¹ und/oder R² einen Halogenalkyl-, Hydroxyalkyl- oder Alkoxyalkylrest mit jeweils bis zu 6 C-Atomen, einen Benzoyloxyalkoxyalkyl-, Acetyloxyalkoxyalkyl- oder Hydroxyalkoxyalkylrest mit jeweils bis zu 11 C-Atomen, eine Acylgruppe mit 1 bis 4 C-Atomen oder R¹ und R² zusammen eine Alkylengruppe mit 2 bis 4 C-Atomen bedeuten, die durch Hydroxy, C₁₋₄-Alkoxy oder Amino substituiert sein kann, X und Y entweder gleich sind und beide jeweils Wasserstoff bedeuten, oder X und Y verschieden sind, wobei einer der Reste X oder Y für Wasserstoff steht und der andere von den beiden Resten eine Hydroxy- oder eine Alkoxygruppe mit bis zu 4 C-Atomen bedeutet,
mit der Maßgabe, daß nicht alle Reste R¹ , R², X und Y gleichzeitig Wasserstoff bedeuten,
sowie deren pharmakologisch unbedenkliche Salze.

2. Verbindungen der allgemeinen Formel I gemäß Anspruch 1 in welchen R¹ und/oder R² Methyl-, Ethyl-, n-Propyl-, Isopropyl- und n-Butyl-, Methoxycarbonylmethylgruppen, Benzylgruppen, 2-Methoxyethylgruppen, Acetylgruppen, ein 2-Aminoethyl-, ein 3-Aminopropyl- oder ein 1-Amino-2-propylrest, ein 2-Dimethylaminoethyl-, ein 3-Dimethylamino-1-propyl-, ein 3-Dimethylamino-2-propyl, ein 2-Diethylaminoethyl-, ein 2-[N-Benzyl-N-methylamino]ethyl-, ein 3-[N-Benzyl-N-methylamino]propyl- oder ein 3-Dimethylamino-2-hydroxy-1-propylrest, ein 3-Diethylamino-2-hydroxy-1-propyl-, ein 3-Piperidino-2-hydroxy-1-propyl, ein 3-Dimethylamino-2-methoxy-1-propyl-, ein 3-Diethylamino-2-methoxyl-1-propyl-, ein 3-Piperidino-2-methoxy-1-propyl-, ein 3-(N-Benzyl-N-methylamino)-2-methoxy-1-propyl-, ein 3-(N-Benzyl-N-methylamino)-2-hydroxy-1-propyl-, ein 3-(N-Methylamino)-2-methoxy-1-propyl-, ein 3-(N-Methylamino)-2-hydroxy-1-propyl-, ein 4-Dimethylamino-3-methoxy-2-butylrest oder eine 2-Piperidinoethyl-, eine 3-Piperidinopropyl, eine 2-Pyrrolidinoethyl- , eine 3-Pyrrolidinopropyl-, eine 2-Morpholinoethyl-, eine 3-Morpholinopropyl-, eine Pyrrolidin2-ylmethyl- oder eine N-Methyl-pyrrolidin-2-ylmethyl-, eine Piperidin-2-ylmethyl-, eine N-Methyl-piperidin-2-ylmethylgruppe oder eine unsubstituierte oder am Stickstoffatom durch C₁₋₄-Alkyl substituierte Piperazinoalkylgruppe mit 1 bis 4 C-Atomen in der Alkylkette oder R¹ und R² zusammen eine unsubstituierte oder hydroxysubstituierte Butylen- oder Propylengruppe bilden, bedeuten.

3. Verfahren zur Herstellung von Indolocarbazol-Derivate der allgemeinen Formel I gemäß Anspruch 1 oder 2 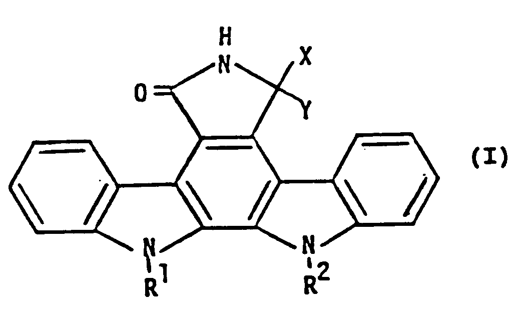 in welcher R¹ und R² gleich oder verschieden sind und Wasserstoff, eine geradkettige oder verzweigte Alkylgruppe mit 1 bis 6 C-Atomen, eine Benzylgruppe, eine unsubstituierte oder substituierte Aminoalkylgruppe mit bis zu 12 C-Atomen, einen Alkoxycarbonylalkylrest mit bis zu 6 C-Atomen, einen Rest -CH₂-CO-NR³R⁴ bei dem R³ und R⁴ gleich oder verschieden sind und für Wasserstoff, eine Alkylgruppe mit 1 bis 4 C-Atomen oder eine Benzylgruppe stehen, oder R¹ und/oder R² einen Halogenalkyl-, Hydroxyalkyl- oder Alkoxyalkylrest mit jeweils bis zu 6 C-Atomen, einen Benzoyloxyalkoxyalkyl-, Acetyloxyalkoxyalkyl- oder Hydroxyalkoxyalkylrest mit jeweils bis zu 11 C-Atomen, eine Acylgruppe mit 1 bis 4 C-Atomen oder R¹ und R² zusammen eine Alkylengruppe mit 2 bis 4 C-Atomen bedeuten, die durch Hydroxy, C₁₋₄-Alkoxy oder Amino substituiert sein kann, X und Y entweder gleich sind und beide jeweils Wasserstoff bedeuten, oder X und Y verschieden sind, wobei einer der Reste X oder Y für Wasserstoff steht und der andere von den beiden Resten eine Hydroxy- oder eine Alkoxygruppe mit bis zu 4 C-Atomen bedeutet,
mit der Maßgabe, daß nicht alle Reste R¹ , R², X und Y gleichzeitig Wasserstoff bedeuten,
sowie deren pharmakologisch unbedenkliche Salze, dadurch gekennzeichnet, daß man
A) Für den Fall, daß X und Y Wasserstoff bedeuten und R¹ und R² gleich sind, jedoch nicht Wasserstoff bedeuten, oder einer der Reste R¹ oder R² für Wasserstoff steht ein Indolocarbazol der Formel II 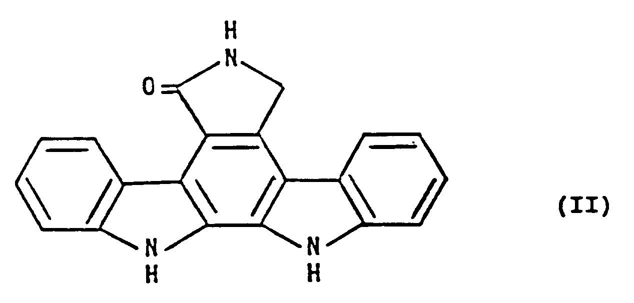 mit einem oder zwei Äquivalenten einer Verbindung der allgemeinen Formel III
R⁵-Z III
in der R⁵ für eine geradkettige oder verzweigte Alkylgruppe mit 1 bis 6 C-Atomen, eine unsubstituierte oder substituierte Aminoalkylgruppe mit bis zu 12 C-Atomen, einen Alkoxycarbonylalkylrest mit bis zu 6 C-Atomen, einen Halogenalkyl- oder Alkoxyalkylrest mit jeweils bis zu 6 C-Atomen, einen Benzoyloxyalkoxyalkyl- oder Acetyloxyalkoxyalkylrest mit jeweils bis zu 11 C-Atomen oder eine Acylgruppe mit 1 bis 4 C-Atomen und Z vorzugsweise für Halogen, insbesondere Jod, Brom und Chlor, oder in der R⁵-Z für ein 1,1-disubstituiertes 3-Hydroxyazetidiniumhalogenid steht,
in Gegenwart von einem oder zwei Äquivalenten einer Base in an sich bekannter Weise an einem oder beiden Indolstickstoffatomen alkyliert oder acyliert, oder indem man bereits eingeführte Reste R⁵ durch Hydrolyse, Etherspaltung, Amidbildung oder Reduktion in die Reste R¹ oder R² überführt, oder
B) daß man für den Fall, daß X und Y Wasserstoff bedeuten, R¹ und R² verschieden sind und weder R¹ noch R² Wasserstoff bedeutet, Verbindungen der allgemeinen Formel I durch Umsetzung von Verbindungen der allgemeinen Formel I, die nach Verfahren A) hergestellt wurden, bei denen entweder R¹ oder R² eine der für R⁵ angegebenen Bedeutungen besitzt und der andere der beiden Reste R¹ oder R² für Wasserstoff steht, mit Verbindungen III, bei denen R⁵ eine andere der für R⁵ angegebenen Bedeutungen besitzt, in Gegenwart von Basen in analoger Weise wie bei Verfahren A) herstellt und gegebenenfalls einer oder beide der Reste mit der Bedeutung von R⁵, wie in Verfahren A) beschrieben, zu einem der Reste R¹ oder R² modifiziert oder
C) daß man für den Fall, daß in der allgemeinen Formel I X oder Y eine Hydroxygruppe oder eine C1-C4-Alkoxygruppe und der andere der beiden Reste Wasserstoff bedeuten, ein Imid der Formel IV 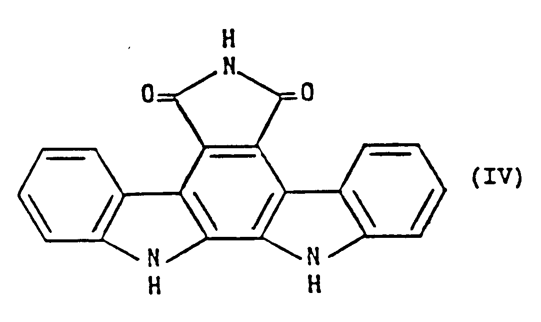 reduziert,
und die so nach den Varianten A, B, oder C erhaltenen Verbindungen I gewünschtenfalls mit Säure in deren pharmakologisch verträgliche Salze überführt.

4. Verfahren zur Herstellung von Verbindungen der allgemeinen Formel I gemäß Anspruch 3 in welchen R¹ und/oder R² Methyl-, Ethyl-, n-Propyl-, Isopropyl- und n-Butyl-, Methoxycarbonyl-methylgruppen, Benzylgruppen, 2-Methoxyethylgruppen, Acetylgruppen, ein 2-Aminoethyl-, ein 3-Aminopropyl- oder ein 1-Amino-2-propylrest, ein 2-Dimethylaminoethyl-, ein 3-Dimethylamino-1-propyl-, ein 3-Dimethylamino-2-propyl, ein 2-Diethylaminoethyl-, ein 2-[N-Benzyl-N-methylamino]ethyl-, ein 3-[N-Benzyl-N-methylamino]propyl- oder ein 3-Dimethylamino-2-hydroxy-1-propylrest, ein 3-Diethylamino-2-hydroxy-1-propyl-, ein 3-Piperidino-2-hydroxy-1-propyl, ein 3-Dimethylamino-2-methoxy-1-propyl-, ein 3-Diethylamino-2-methoxy-1-propyl-, ein 3-Piperidino-2-methoxy-1-propyl-, ein 3-(N-Benzyl-N-methylamino)-2-methoxy-1-propyl-, ein 3-(N-Benzyl-N-methylamino)-2-hydroxy-1-propyl-, ein 3-(N-Methylamino)-2-methoxy-1-propyl-, ein 3-(N-Methylamino)-2-hydroxy-1-propyl-, ein 4-Dimethylamino-3-methoxy-2-butylrest oder eine 2-Piperidinoethyl-, eine 3-Piperidinopropyl, eine 2- Pyrrolidinoethyl- , eine 3-Pyrrolidinopropyl-, eine 2-Morpholinoethyl-, eine 3-Morpholinopropyl, eine Pyrrolidin-2-ylmethyl- oder eine N-Methyl-pyrrolidin-2-ylmethyl-, eine Piperidin-2-ylmethyl-, eine N-Methyl-piperidin-2-ylmethylgruppe oder eine unsubstituierte oder am Stickstoffatom durch C₁₋₄-Alkyl substituierte Piperazinoalkylgruppe mit 1 bis 4 C-Atomen in der Alkylkette, oder R¹ und R² zusammen eine unsubstituierte oder hydroxysubstituierte Butylen- oder Propylengruppe, bilden, bedeuten.

5. Verwendung von Verbindungen der allgemeinen Formel 1 gemäß Ansprüche 1 bis 2 zur Herstellung von Arzneimitteln zur Behandlung von Herz- und Gefäßkrankheiten wie Thrombosen, Arteriosklerose, Hypertension, von Entzündungsprozessen, Allergien, Krebs und bestimmten degenerativen Schäden des Zentralnervensystems.

6. Arzneimittel mit üblichen Hilfs- und Trägerstoffen enthaltend mindestens eine Verbindung nach Anspruch 1 als Wirkstoff.

## Claims

1. Indolocarbazole derivatives of the general formula I: 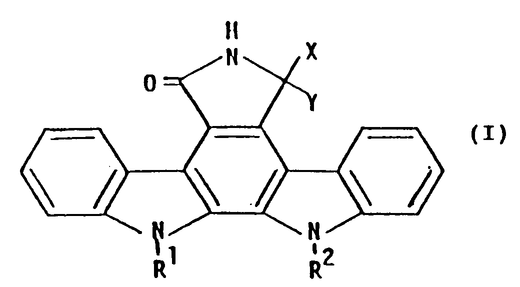 wherein R¹ and R², which can be the same or different, are hydrogen atoms, straight-chained or branched alkyl radicals containing 1 to 6 carbon atoms, benzyl radicals, unsubstituted or substituted aminoalkyl radicals containing up to 12 carbon atoms, alkoxycarbonylalkyl radicals containing up to 6 carbon atoms, -CH₂-CO-NR³R⁴ radicals, wherein R³ and R⁴ are the same or different and are hydrogen atoms, alkyl radicals containing 1 to 4 carbon atoms or benzyl radicals, or R¹ and/or R² are haloalkyl, hydroxyalkyl or alkoxyalkyl radicals containing, in each case, up to 6 carbon atoms, benzoyloxyalkoxyalkyl, acetyloxyalkoxyalkyl or hydroxyalkoxyalkyl radicals with, in each case, up to 11 carbon atoms, or acyl radicals containing 1 to 4 carbon atoms, or R¹ and R² together form an alkylene radical containing 2 to 4 carbon atoms, which can optionally be substituted by hydroxyl, C₁₋₄-alkoxy or amino radicals, and wherein X and Y are either the same and signify hydrogen atoms or are different, one of them being a hydrogen atom and the other being a hydroxyl group or an alkoxy radical containing up to 4 carbon atoms, with the proviso that not all of R¹, R², X and Y simultaneously stand for hydrogen atoms; as well as the pharmacologically acceptable salts thereof.

2. Compounds of the general formula I according to claim 1 wherein R¹ and/or R² are methyl, ethyl, n-propyl, isopropyl or n-butyl radicals, methoxycarbonylmethyl radicals, benzyl radicals, 2-methoxyethyl radicals, acetyl radicals, a 2-aminoethyl, 3-aminopropyl or 1-amino-2-propyl radical, a 2-dimethylaminoethyl, 3-dimethylamino-1-propyl, 3-dimethylamino-2-propyl, 2-diethylaminoethyl, 2-[N-benzyl-N-methylamino]-ethyl, 3-[N-benzyl-N-methylamino]-propyl or 3-dimethylamino-2-hydroxy-1-propyl radical, a 3-diethylamino-2-hydroxy-1-propyl, 3-piperidino-2-hydroxy-1-propyl, 3-dimethylamino-2-methoxy-1-propyl, 3-diethylamino-2-methoxy-1-propyl, 3-piperidino-2-methoxy-1-propyl, 3-(N-benzyl-N-methylamino)-2-methoxy-1-propyl, 3-(N-benzyl-N-methylamino)-2-hydroxy-1-propyl, 3-(N-methylamino)-2-methoxy-1-propyl, 3-(N-methylamino)-2-hydroxy-1-propyl or 4-dimethylamino-3-methoxy-2-butyl radical or a 2-piperidinoethyl, 3-piperidinopropyl, 2-pyrrolidinoethyl, 3-pyrrolidinopropyl, 2-morpholinoethyl, 3-morpholinopropyl, pyrrolidin-2-ylmethyl or an N-methyl-pyrrolidin-2-ylmethyl, a piperidin-2-ylmethyl, an N-methyl-piperidin-2-ylmethyl radical or a piperazinoalkyl radical with 1 to 4 carbon atoms in the alkyl chain, unsubstituted or substituted at the nitrogen atom by C₁₋₄-alkyl, or R¹ and R² together form an unsubstituted or hydroxy-substituted butylene or propylene radical.

3. Process for the preparation of indolocarbazole derivatives of the general formula I, according to claim 1 or claim 2, 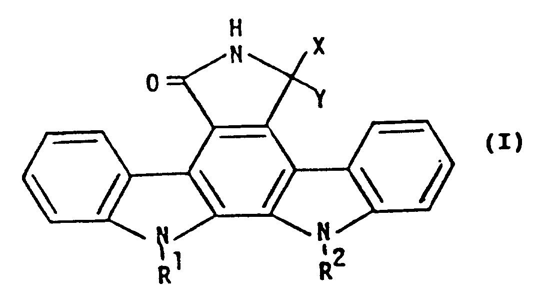 wherein R¹ and R², which can be the same or different, are hydrogen atoms, straight-chained or branched alkyl radicals containing 1 to 6 carbon atoms, benzyl radicals, unsubstituted or substituted aminoalkyl radicals containing up to 12 carbon atoms, alkoxycarbonylalkyl radicals containing up to 6 carbon atoms, -CH₂-CO-NR³R⁴ radicals, wherein R³ and R⁴ are the same or different and are hydrogen atoms, alkyl radicals containing 1 to 4 carbon atoms or benzyl radicals, or R¹ and/or R² are haloalkyl, hydroxyalkyl or alkoxyalkyl radicals containing, in each case, up to 6 carbon atoms, benzoyloxyalkoxyalkyl, acetyloxyalkoxyalkyl or hydroxyalkoxyalkyl radicals with, in each case, up to 11 carbon atoms, or acyl radicals containing 1 to 4 carbon atoms, or R¹ and R² together form an alkylene radical containing 2 to 4 carbon atoms, which can optionally be substituted by hydroxyl, C₁₋₄-alkoxy or amino radicals, and wherein X and Y are either the same and signify hydrogen atoms or are different, one of them being a hydrogen atom and the other being a hydroxyl group or an alkoxy radical containing up to 4 carbon atoms, with the proviso that not all of R¹, R², X and Y simultaneously stand for hydrogen atoms; as well as the pharmacologically acceptable salts thereof,
characterised in that
A) when X and Y are hydrogen atoms and R¹ and R², are the same but are not hydrogen atoms, or when one of R¹ and R² is a hydrogen atom, an indolocarbazole of the formula II: 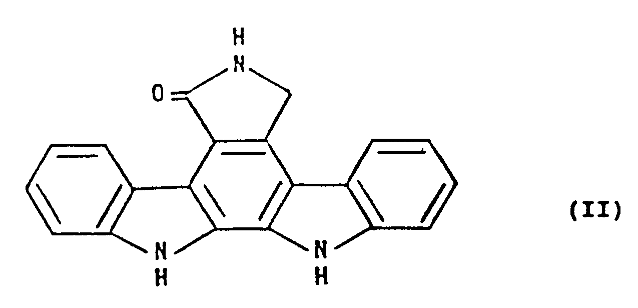 is alkylated or acylated in known manner on one or both indole nitrogen atoms in the presence of one or two equivalents of base with one or two equivalents of a compound of the general formula III:
R⁵ - Z (III)
wherein R⁵ is a straight-chained or branched alkyl radical containing 1 to 6 carbon atoms, an unsubstituted or substituted aminoalkyl radical containing up to 12 carbon atoms, an alkoxycarbonylalkyl radical containing up to 6 carbon atoms, a haloalkyl or alkoxyalkyl radical with, in each case, up to 6 carbon atoms, a benzoylalkoxyalkyl or acetyloxyalkyl radical with, in each case, up to 11 carbon atoms, or an acyl radical containing 1 to 4 carbon atoms and Z is preferably a halogen atom, especially an iodine, bromine or chlorine atom, or wherein R⁵ - Z is a 1,1-disubstituted 3-hydroxyazetidinium halide, or by converting a previously introduced radical R⁵ by hydrolysis, ether cleavage, amide formation or reduction into a radical R¹ or R², or
B) when X and Y are both hydrogen atoms and R¹ and R² are different and neither R¹ nor R² is a hydrogen atom, compounds of general formula I are prepared by reacting a compound of general formula I, which has been prepared according to process A) and in which either R¹ or R² has one of the meanings given for R⁵ and the other is a hydrogen atom, is reacted with a compound III in which R⁵ has another of the meanings given for R⁵, in the presence of a base in a manner analogous to that described for process A) and, if desired, one or both of the radicels with the meaning of R⁵ is modified, as described in process A), to give one of the radicals R¹ or R²; or
C) when, in the general formula I, X or Y is a hydroxyl group or a C₁-C₄-alkoxy group and the other is a hydrogen atom, an imide of the formula IV: 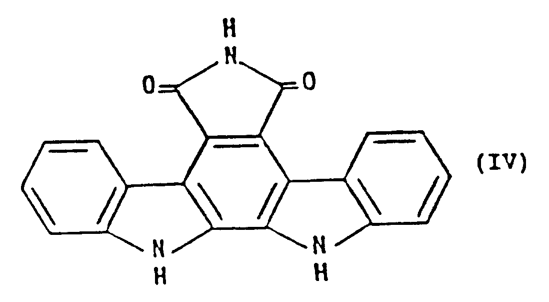 is reduced,
and the compound of general formula I obtained by process variants A), B) or C) is, if desired, converted with an acid into a pharmacologically acceptable salt.

4. Process for the preparation of compounds of general formula I according to claim 3 wherein R¹ and/or R² are methyl, ethyl, n-propyl, isopropyl or n-butyl radicals, methoxycarbonylmethyl radicals, benzyl radicals, 2-methoxyethyl radicals, acetyl radicals, a 2-aminoethyl, 3-aminopropyl or 1-amino-2-propyl radicals, a 2-dimethylaminoethyl, 3-dimethylamino-1-propyl, 3-dimethylamino-2-propyl, 2-diethylaminoethyl, 2-[N-benzyl-N-methylamino]-ethyl, 3-[N-benzyl-N-methylamino]-propyl or 3-dimethylamino-2-hydroxy-1-propyl radical, a 3-diethylamino-2-hydroxy-1-propyl, 3-piperidino-2-hydroxy-1-propyl,3-dimethylamino-2-methoxy-1-propyl, 3-diethylamino-2-methoxy-1-propyl, 3-piperidino-2-methoxy-1-propyl, 3-(N-benzyl-N-methylamino)-2-methoxy-1-propyl, 3-(N-benzyl-N-methylamino)-2-hydroxy-1-propyl, 3-(N-methylamino)-2-methoxy-1-propyl, 3-(N-methylamino)-2-hydroxy-1-propyl or 4-dimethylamino-3-methoxy-2-butyl radical or a 2-piperidinoethyl, 3-piperidinopropyl, 2-pyrrolidinoethyl, 3-pyrrolidinopropyl, 2-morpholinoethyl, 3-morpholinopropyl, pyrrolidin-2-ylmethyl or an N-methyl-pyrrolidin-2-ylmethyl, a piperidin-2-ylmethyl, an N-methyl-piperidin-2-ylmethyl radical or a piperazinoalkyl radical with 1 to 4 carbon atoms in the alkyl chain, unsubstituted or substituted at the nitrogen atom by C₁₋₄-alkyl, or R¹ and R² together form an unsubstituted or hydroxy-substituted butylene or propylene radical.

5. Use of compounds of general formula I according to claims 1 to 2 for the manufacture of pharmaceutical compositions for the treatment of heart and blood vessel diseases, such as thromboses, arteriosclerosis, hypertension, inflammatory processes, allergies, cancers and certain degenerative damage of the central nervous system.

6. Pharmaceutical compositions with conventional adjuvant and carrier materials containing at least one compound according to claim 1 as active material.

## Revendications

1. Dérivé d'indolocarbazol de formule générale I 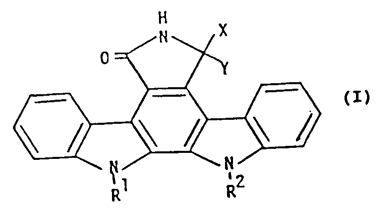 ainsi que ses sels pharmacologiques neutres,
formule dans laquelle R¹ et R² sont identiques ou différents et représentent un atome d'hydrogène, un groupe alkyle à chaîne linéaire ou ramifiée de 1 à 6 atomes de carbone, un groupe benzyle, un groupe aminoalkyle substitué ou non substitué avec jusqu'à 12 atomes de carbone, un résidu alkoxycarbonylalkyle avec jusqu'à 6 atomes de carbone, un résidu -CH₂-CO-NR³R⁴ dans lequel R3 et R4 sont identiques ou différents et représentent un atome d'hydrogène, un groupe alkyle de 1 à 4 atomes de carbone, ou un groupe benzyle, ou R1 et/ou R2 représentent un résidu halogénoalkyle, hydroxyalkyle ou alkoxyalkyle, lesquels peuvent comporter jusqu'à 6 atomes de carbone, un résidu benzoyloxyalkoxyalkyle, acétyloxyalkoxyalkyle ou hydroxyalkoxyalkyle lesquels peuvent comporter jusqu'à 11 atomes de carbone, un groupe acyle de 1 à 4 atomes de carbone, où R¹ et R² ensemble représentent un groupe alkylène de 2 à 4 atomes de carbone, qui peut être substitué par un groupe hydroxy, alkoxy en C_{1-C4} ou amino, X et Y sont soit identiques et représentent alors tous deux un atome d'hydrogène, soit ils sont différents et dans ce cas, un des résidus X ou Y représente un atome d'hydrogène et l'autre représente un groupe hydroxy ou un groupe alkoxy contenant jusqu'à 4 atomes de carbone, avec cette réserve que les résidus R¹, R², X et Y ne peuvent simultanément consister en un atome d'hydrogène.

2. Compositions de formule générale I selon la revendication 1, dans lesquelles R1 et/ou R2 représentent des groupes méthyl,-éthyle, -n-propyle, isopropyle et n-butyle, méthoxycarbonylméthyl, benzyle, 2-méthoxyéthyle, acétyle, des résidus 2-aminoéthyle, 3-aminopropyle ou 1-amino-2-propyle, des résidus 2-diméthylaminoéthyle, 3-diméthylamino-1-propyle, 3-dimethylamino-2-propyle, 2-diéthylaminoéthyle, des résidus 2-[N-benzyl-N-méthylamino]-éthyle, des résidus 3-[N-benzyle-N-méthyl-amino]-propyle ou 3-diméthylamino-2-hydroxy-1-propyle, des résidus 3-diéthyl-amino-2-hydroxy-1-propyle, 3-piperidino-2-hydroxy-1-propyle, 3-dimethyl-amino-2-méthoxy-1-propyle, 3-diéthylamino-2-méthoxy-1-propyle, 3-piperidino-2-méthoxy-1-propyle, 3-(N-benzyle-n-méthylamino)-2-méthoxy-1-propyle, 3-(N-benzyl-N-méthylamino)-2-hydroxy-1-propyle, 3-(N-méthylamino)-2-méthoxy-1-propyle, 3-(N-méthylamino)-2-hydroxy-1-propyle, 4-diméthylamino-3-méthoxy-2-butyle ou les groupes 2-pipéridinoéthyle, 3-pipéridinopropyle, 2-pyrrolidinoéthyle, 3-pyrrolidinopropyle, 2-morpholino-éthyle, 3-morpholino-propyle, pyrrolidine-2-ylméthyle ou un groupe N-méthyl-pyrrolidine-2-ylméthyle, une groupe pipéridine-2-ylméthyle, un groupe N-méthyl-pipéridine-2-ylméthyle ou un groupe pipérazinoalkyle non substitué ou substitué sur l'atome d'azote par un groupe alkyle en C₁₋₄ présentant de 1 à 4 atomes de carbone dans la chaîne alkyle ou R¹ et R² constituent ensemble un groupe butylène ou un groupe propylène qui sont non substitués ou hydroxysubstitués.

3. Procédé de préparation d'un dérivé d'indocarbazole de formule générale I selon la revendication 1 ou la revendication 2 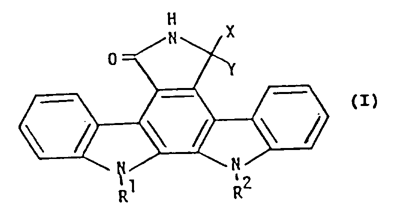 ainsi que leurs sels pharmacologiques neutres,
formule dans laquelle R¹ et R² sont identiques ou différents et représentent un atome d'hydrogène, un groupe alkyle à chaîne linéaire ou ramifiée de 1 à 6 atomes de carbone, un groupe benzyle, un groupe aminoalkyle substitué ou non substitué avec jusqu'à 12 atomes de carbone, un résidu alkoxycarbonylalkyle avec jusqu'à 6 atomes de carbone, un résidu -CH₂-CO-NR³R⁴ dans lequel R3 et R4 sont identiques ou différents et représentent un atome d'hydrogène, un groupe alkyle de 1 à 4 atomes de carbone, ou un groupe benzyle, ou R1 et/ou R2 représentent un résidu halogénoalkyle, hydroxyalkyle ou alkoxyalkyle, lesquels peuvent comporter avec à chaque fois jusqu'à 6 atomes de carbone, un résidu benzoyloxyalkoxyalkyle, acétyloxyalkoxyalkyle ou hydroxyalkoxyalkyle lesquels peuvent comporter jusqu'à 11 atomes de carbone, un groupe acyle de 1 à 4 atomes de carbone, où R¹ et R² ensemble représentent un groupe alkylène de 2 à 4 atomes de carbone, qui peut être substitué par un groupe hydroxy, alkoxy en C₁-C₄ ou amino, X et Y sont soit identiques et représentent alors tous deux un atome d'hydrogène, soit ils sont différents et dans ce cas, un des résidus X ou Y représente un atome d'hydrogène et l'autre représente un groupe hydroxy ou un groupe alkoxy avec jusqu'à 4 atomes de carbone, avec cette réserve que les résidus R¹, R², X et Y ne peuvent simultanément consister en un atome d'hydrogène, caractérisé en ce que
(A) dans le cas où X et Y représentent un atome d'hydrogène et R¹ et R² sont identiques mais ne représentent pas un atome d'hydrogène, ou bien où l'un des résidus R¹ ou R² représente un atome d'hydrogène, on transforme un indolocarbazole de formule II 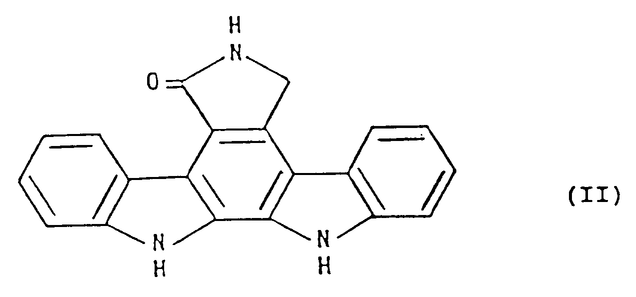 avec un ou deux équivalents d'une composition de formule générale III
R⁵-Z III
dans laquelle:
- R⁵ représente un groupe alkyle à chaîne linéaire ou ramifiée, avec de 1 à 6 atomes de carbone, un groupe aminoalkyl non substitué ou substitué avec jusqu'à 12 atomes de carbone, un résidu alkoxycarbonylalkyle avec jusqu'à 6 atomes de carbone, un résidu halogénure d'alkyle ou alkoxyalkyle, lesquels peuvent comporter jusqu'à 6 atomes de carbone, un résidu benzoyloxyalkoxyalkyl ou acétyloxyalkoxyalkyle e.p.c. jusqu'à 11 atomes de carbone, ou un groupe acyle de 1 à 4 atomes de carbone, et
- Z représente de préférence un atome d'halogène, en particulier iode, brome et chlore,
- ou R⁵-Z représente un 3-halogénure de 3-hydroxyazétitinium1,1-disubstitué, et alkyle ou acylé
en présence de 1 ou 2 équivalent d'une base d'une façon connue alkylé ou acylé sur un ou les deux atomes d'azote de l'indole, ou par transformation des résidus R⁵ déjà introduits par hydrolyse, clivage de l'éther, formation d'amide ou réduction en les résidus R¹ ou R², ou
(B) dans les cas où X et Y représentent des atomes d'hydrogène et R¹ et R² sont différents, et ni R¹ ni R² ne représentent un atome d'hydrogène, par transformation des compositions de formule générale I, préparées selon le procédé A dans lesquelles soit R¹ ou R² représentent une des significations attribuées à R⁵, et l'autre des deux résidus R¹ ou R² représente un atome d'hydrogène, avec la composition III, dans laquelle R⁵ représente une autre des significations attribuées à R⁵, en présence de bases de façon analogue au procédé A), et le cas échéant un ou les deux résidus ayant les significations attribuées à R⁵, comme décrit dans le procédé A) est(sont) modifiée(s) en un des résidus R¹ ou R², ou
(C) dans le cas où dans la formule générale 1, l'un des substituants X ou Y représente un groupe hydroxy ou un groupe alkoxy en C₁-C₄ et l'autre des deux résidus représente un atome d'hydrogène 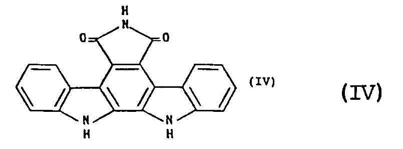
un imide de formule IV est réduit, et la composition I obtenue ainsi selon les variantes A, B ou C est transformée de préférence avec des acides, en sels pharmacologiquement acceptables.

4. Procédé de préparation de composition de formule générale I selon la revendication 3, dans lesquelles R1 et/ou R2 représentent des groupes méthyl-éthyle, -n-propyle, isopropyle et n-butyle, méthoxycarbonylméthyl, benzyle, 2-méthoxyéthyle, acétyle, des résidus 2-aminoéthyle, 3-aminopropyle ou 1-amino-2-propyle, des résidus 2-diméthylaminoéthyle, 3-diméthylamino-1-propyle, 3-diméthylamino-2-propyle, 2-diéthylaminoéthyle, des résidus 2-[N-benzyl-N-méthylamino]éthyle, des résidus 3-[N-benzyle-N-méthylamino]propyle ou 3-diméthylamino-2-hydroxy-1-propyle, des résidus 3-diéthylamino-2-hydroxy-1-propyle, 3-piperidino-2-hydroxy-1-propyle, 3-diméthyl-amino-2-méthoxy-1-propyle, 3-diéthylamino-2-méthoxy-1-propyle, 3-piperidino-2-méthoxy-1-propyle, 3-(N-benzyle-n-méthylamino)-2-méthoxy-1-propyle, 3-(N-benzyl-N-méthyl-amino)-2-hydroxy-1-propyle, 3-(N-méthylamino)-2-méthoxy-1-propyle, 3-(N-méthylamino)-2-hydroxy-1-propyle, 4-diméthylamino-3-méthoxy-2-butyle ou les groupes 2-pipéridinoéthyle, 3-pipéridinopropyle, 2-pyrrolidinoéthyle, 3-pyrrolidinopropyle, 2-morpholinoéthyle, 3-morpholinopropyle, pyrrolidine-2-ylméthyle ou un groupe N-méthyl-pyrrolidine-2-ylméthyle, une groupe pipéridine-2-ylméthyle, un groupe N-méthyl-pipéridine-2-ylméthyle ou un groupe pipérazinoalkyle non substitué ou substitué sur l'atome d'azote par un groupe alkyle en C₁₋₄ présentant de 1 à 4 atomes de carbone dans la chaîne alkyle où R¹ et R² constituent ensemble un groupe butylène ou un groupe propylène qui sont non substitués ou hydroxysubstitués.

5. Utilisation des compositions de formule générale I selon les revendications 1 et 2 pour la préparation de médicaments destinés au traitement de maladies cardio-vasculaires, telles que thromboses, artérioscléroses, hypertension artérielle, d'inflammations, d'allergies, de cancers et de certaines maladies dégénératives du système nerveux.

6. Médicament comportant des matériaux supports et des adjuvants classiques et comprenant au moins une composition selon la revendication 1 en tant que matière active.
